(19)

Europäisches
Patentamt
European
Patent Office
Office européen
des brevets

(11) **EP 4 506 465 A1**

(12) **EUROPEAN PATENT APPLICATION**
published in accordance with Art. 153(4) EPC

(43) Date of publication:
**12.02.2025 Bulletin 2025/07**

(21) Application number: **22859528.6**

(22) Date of filing: **04.08.2022**

(51) International Patent Classification (IPC):
*C12Q 1/6827* (2018.01)      *C12Q 1/6886* (2018.01)

(52) Cooperative Patent Classification (CPC):
**G16B 20/20; C12Q 1/68; C12Q 1/6806;**
**C12Q 1/6827; C12Q 1/6844; C12Q 1/6869;**
**C12Q 1/6886; G16B 30/00; G16B 30/10**

(86) International application number:
**PCT/KR2022/011529**

(87) International publication number:
**WO 2023/182586 (28.09.2023 Gazette 2023/39)**

(84) Designated Contracting States:
**AL AT BE BG CH CY CZ DE DK EE ES FI FR GB**
**GR HR HU IE IS IT LI LT LU LV MC MK MT NL NO**
**PL PT RO RS SE SI SK SM TR**
Designated Extension States:
**BA ME**
Designated Validation States:
**KH MA MD TN**

(30) Priority:  **21.03.2022  KR 20220034590**
          **30.06.2022  KR 20220080571**

(71) Applicant: **IMB DX, Inc.**
**Geumcheon-gu**
**Seoul 08506 (KR)**

(72) Inventors:
• **ROH, Han Seong**
**Seoul 07015 (KR)**
• **KIM, Su Yeon**
**Daejeon 34191 (KR)**
• **KIM, Hwang Phill**
**Yongin-si, Gyeonggi-do 16990 (KR)**
• **MOON, Sung Tae**
**Seoul 05224 (KR)**
• **KIM, Tae You**
**Seoul 03009 (KR)**

(74) Representative: **Cabinet Nony**
**11 rue Saint-Georges**
**75009 Paris (FR)**

(54) **METHOD FOR DISCRIMINATING FALSE POSITIVE VARIANT DURING NUCLEIC ACID ANALYSIS**

(57)    The present invention relates to a method of identifying false positive variants during nucleic acid sequencing. According to one embodiment of the present invention, it is possible to effectively identify and remove false positive variants using the context error rate of HQS obtained from an amplified nucleic acid fragment.

Fig. 1

EP 4 506 465 A1

**Description**

**Technical Field**

The present invention relates to a method of identifying false positive variants during nucleic acid sequencing.

**Background Art**

Detection of somatic variants from large amounts of sequencing data is critical to genomic research. In recent sequencing technology, the accuracy of detection of somatic variants, such as the detection of low-frequency somatic variants or the identification of genetic subclones in a sample, has been significantly improved. These technological advances have increased opportunities for clinical application of high-efficiency next-generation sequencing in diagnosing genetic diseases that are difficult to detect by general clinical trials or in diagnosing cancers from liquid biopsies.

In recent years, cell-free DNA (cfDNA) or circulating tumor DNA (ctDNA) present in blood has been used to detect cancer. In healthy individuals, most of the DNA is released from hematopoietic cells, but in cancer patients, cfDNA contains ctDNA released from dying tumor cells into the blood. This ctDNA contains genetic mutations related to cancer, and monitoring of these genetic mutations enables early detection of cancer before the occurrence of lesions, analysis of responses to specific cancer treatments, discovery of mechanisms for generating resistance to anticancer drugs, detection of the presence of residual cancer, and the like.

Meanwhile, cfDNA mixed with hematopoietic cell-derived DNA is present in blood, but since the fraction of cfDNA is less than 1% of the total in many cases, significant levels of false positives or false negatives still occur in the analysis process. As techniques for solving this problem, techniques such as molecular barcoding techniques, digital error suppression methods, and annotation-based filtering techniques have been reported, but they have not yet been commercialized for use in clinical diagnosis.

Accordingly, the present invention is intended to propose a technique capable of identifying and removing false positive variants with high accuracy based on context error rate information for HQS obtained from an amplified nucleic acid fragment.

**DISCLOSURE**

**Technical Problem**

An object of one aspect of the present invention is to provide a method of identifying false positive variants in nucleic acid sequencing, the method comprising steps of: a) extracting a nucleic acid fragment containing a candidate variant from a target sample; b) adding a unique molecular identifier (UMI) to the end of the extracted nucleic acid fragment; c) producing a high-quality unique sequence (HQS) by amplifying the nucleic acid fragment to which the UMI has been added; d) obtaining an LLRc value by applying an error rate corresponding to the HQS; and e) determining the presence or absence of a false positive variant from the LLRc value.

**Technical Solution**

One aspect of the present invention provides a method of removing false positive variants in nucleic acid sequencing, the method comprising steps of:

a) extracting a nucleic acid fragment containing a candidate variant from a target sample;
b) adding a unique molecular identifier (UMI) to the end of the extracted nucleic acid fragment;
c) producing a high-quality unique sequence (HQS) by amplifying the nucleic acid fragment to which the UMI has been added;
d) obtaining an LLRc value according to the following Equation I by applying an error rate corresponding to the HQS; and
e) determining the presence or absence of a false positive variant from the LLRc value:

[Equation I]

$$LLR_C = \sum_{r=1}^{N} ln\left(\frac{f \times (1-H) + (1-f) \times H}{H}\right) \qquad H = \begin{cases} e_{r(S),C}, & if\ r = alt\ read \\ 1 - e_{r(S),C}, & otherwise \end{cases}$$

wherein r denotes read, N denotes the total number of reads, S denotes family size, f denotes variant allele frequency, and e denotes error rate.

In one embodiment of the present invention, the candidate variant may be at least one selected from the group consisting of single-nucleotide variation, nucleotide insertion, and nucleotide deletion.

In one embodiment of the present invention, the HQS in step c) may be a single-strand consensus sequence (SSCS) or a duplex consensus sequence (DCS).

In one embodiment of the present invention, the family size may be 2 to 30.

In one embodiment of the present invention, the error rate may include all of a context error rate at a specific family size, a nucleotide error rate calculated in an error correction process, and a read error rate calculated in a mapping process.

In one embodiment of the present invention, step e) of determining the presence or absence of a false positive variant may comprise: obtaining a weighted LLR value by calculating an LLR value for SSCS and an LLR value for DCS; and determining that, when a cut-off value set using a precision-recall curve from the weighted LLR value is 50 or more, the variant in the nucleic acid fragment containing the candidate variant is false positive.

**Advantageous Effects**

According to one embodiment of the present invention, it is possible to effectively identify and remove false positive variants using the context error rate versus the size of a nucleic acid fragment family obtained from an amplified nucleic acid fragment.

**Brief Description of Drawings**

FIG. 1 is a diagram showing the occurrence of errors by PCR amplification of a UMI-added nucleic acid fragment and the family size resulting from amplification of the nucleic acid fragment.

FIG. 2 shows context error types and an error rate for each error type.

FIG. 3 shows a context error rate for each family size.

FIG. 4 depicts color-coded diagrams showing context error rates for each family size in SSCS and DCS.

FIG. 5 depicts graphs showing error rates of SSCS and DCS in a family size of 2 to 15.

FIG. 6 depicts graphs showing the error rate versus family size in deletion contexts.

FIG. 7 depicts graphs showing the error rate versus family size in insertion contexts.

FIG. 8 depicts graphs showing the results of setting variant score cutoffs using precision-recall graphs in SNV, insertion and deletion contexts.

**Best Mode**

One aspect of the present invention provides a method of identifying false positive variants in nucleic acid sequencing, the method comprising steps of:

a) extracting a nucleic acid fragment containing a candidate variant from a target sample;

b) adding a unique molecular identifier (UMI) to the end of the extracted nucleic acid fragment;

c) producing a high-quality unique sequence (HQS) by amplifying the nucleic acid fragment to which the UMI has been added;

d) obtaining an LLRc value according to the following Equation I by applying an error rate corresponding to the HQS; and

e) determining the presence or absence of a false positive variant from the LLRc value:

[Equation I]

$$LLR_C = \sum_{r=1}^{N} ln\left(\frac{f \times (1-H) + (1-f) \times H}{H}\right) \qquad H = \begin{cases} e_{r(s),c}, & if\ r = alt\ read \\ 1 - e_{r(s),c}, & otherwise \end{cases}$$

wherein r denotes read, N denotes the total number of reads, S denotes family size, f denotes variant allele frequency, and e denotes error rate.

It is known that, in the blood of cancer patients, primary cancer-derived circulating tumor DNA (ctDNA) and cell-free DNA (cfDNA) mixed with hematopoietic cell-derived DNA circulate together. In particular, it is known that the amounts of the DNAs are larger in cancer patients than in healthy individuals and differs between before and after chemotherapy, and when cancer recurs after treatment, the amount of ctDNA increases. The present inventors have performed somatic variant analysis for cfDNA in the process of diagnosing cancer from liquid biopsy, and conducted studies to identify false positive variants, which are generated during the analysis process, with high accuracy and sensitivity. As a result, the present inventors have found that false positive variants can be effectively identified using the context error rate versus the family size of the amplified nucleic acid fragments, thereby completing the present invention.

Hereinafter, a method of identifying false-positive variants in nucleic acid sequencing according to the present invention will be described in detail.

First, in the method of the present invention, step a) of extracting a nucleic acid fragment containing a candidate variant from a target sample is performed.

As used herein, the term "sample" is meant to include, but not limited to, samples such as tissue, cells, whole blood, serum, plasma, saliva, sputum, cerebrospinal fluid or urine, from which a material for targeted sequencing can be obtained to analyze a variant in a nucleotide sequence. Preferably, the sample may be serum or plasma.

In step a), the nucleic acid may be a genome or a fragment thereof. As used herein, the term "genome" refers to the entirety of chromosomes, chromatin, or genes. The genome or fragment thereof may be an isolated DNA, such as cell-free nucleic acid (cfDNA). The method of extracting or isolating nucleic acid from the target sample may be performed by a method known to those skilled in the art.

The nucleic acid fragment is interpreted as a concept including fragmentation of the extracted nucleic acid. Fragmentation refers to a process in which a genome is naturally degraded while circulating in the blood, or is artificially cleaved physically, chemically, or enzymatically. Through this process, nucleic acid fragments having various lengths and reads at both ends thereof may be produced. As used herein, the term "read" refers to sequence information for one or more nucleic acid fragments generated from nucleic acid sequencing, and the length of the nucleic acid fragment may be calculated using reads at both ends of the nucleic acid fragment. The length of the nucleic acid fragment may be about 10 bp to about 2,000 bp, preferably about 50 bp to about 500 bp.

According to one embodiment of the present invention, the candidate variant may preferably be a somatic variant, a single nucleotide variation (SNV) in a sequencing read, nucleotide insertion, or nucleotide deletion, without being limited thereto.

In single nucleotide variations, each of four nucleotides can change into three different nucleotides, and thus a total of 12 single nucleotide variations exist. Accordingly, error rates can be observed due to a variety of causes, such as biological causes, and mechanical errors appearing in the entire experimental process starting from the extraction of nucleic acids and in the sequencing process.

Next, step b) of adding a unique molecular identifier to the end of the extracted nucleic acid fragment is performed.

As used herein, the term "unique molecular identifier (UMI)" refers to a sequence consisting of 4- to 10-bp DNA, which is a barcode sequence that binds to the end of a nucleic acid fragment and labels the nucleic acid fragment.

Using the unique molecular identifier, when the nucleic acid fragment is duplicated by PCR amplification, it is possible to distinguish different nucleic acid fragments, and using this, it is possible to correct errors by comparing PCR duplicates with each other.

For example, when UMIs having different sequences are added to both directions of a nucleic acid fragment, it is possible to identify which strand of double strands is a PCR amplification product (duplex sequencing).

Next, step c) of generating HQS by amplifying the nucleic acid fragment to which the UMI has been added is performed.

The amplification of the UMI-added nucleic acid fragment refers to the polymerase chain reaction (PCR) involved in the next-generation sequencing (NGS) process, and random sequencing errors can occur with a certain frequency during the PCR process.

As used herein, the term "'family" refers to duplicates produced as the nucleic acid fragment to which the UMI has been added is amplified in the PCR process, and the term "family size" refers to the number of members of the family.

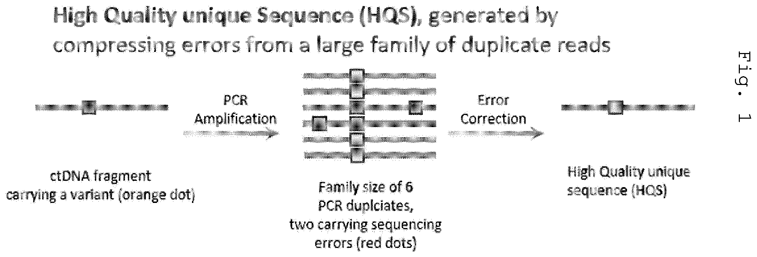

FIG. 1 shows that UMI-added nucleic acid fragments can cause errors by PCR amplification (red dots), and shows an example of a family size resulting from amplification of the nucleic acid fragment.

According to one embodiment of the present invention, the HQS may be a single-strand consensus sequence (SSCS) or a duplex consensus sequence (DCS).

In the process of producing the single-strand consensus sequence (SSCS), PCR duplicates generated from DNA strands in one direction are compared to each other to correct erroneous nucleotides, but there is a limitation in that errors generated in the initial stage prior to PCR are continuously accumulated during the PCR process.

In the process of producing the duplex consensus sequence (DCS), SSCSs in both directions are compared with each other to obtain a consensus sequence, and thus errors generated physically and chemically in the initial stage prior to PCR are compared and those with a difference are considered errors and removed.

Then, step d) of obtaining an LLRc value according to the following Equation I by applying an error rate corresponding to the HQS is performed.

[Equation I]

$$LLR_C = \sum_{r=1}^{N} ln\left(\frac{f \times (1-H) + (1-f) \times H}{H}\right) \quad H = \begin{cases} e_{r(S),C}, & if\ r = alt\ read \\ 1 - e_{r(S),C}, & otherwise \end{cases}$$

wherein r denotes read, N denotes the total number of reads, S denotes family size, f denotes variant allele frequency, and e denotes error rate.

As used herein, the term "error rate" refers to the multiplier of the family size for the error probability of a specific nucleotide. For example, assuming that the error probability of a certain nucleotide is x and the family size is n, the error rate may be x^n. Thus, as the family size increases, a more accurate consensus sequence can be obtained.

According to one embodiment of the present invention, the family size may be 2 to 30, preferably 2 to 15, more preferably 2 to 10, most preferably 2 to 7.

According to one embodiment of the present invention, the error rate may include all of a context error rate at a specific family size, a nucleotide error rate calculated in an error correction process, and a read error rate calculated in a mapping process.

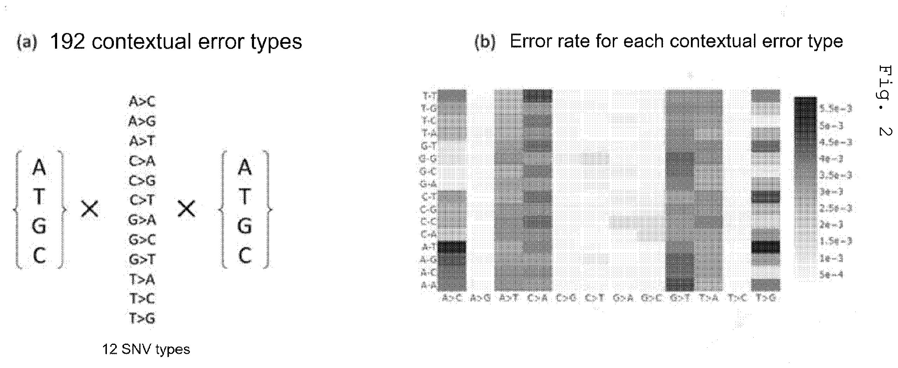

As used herein, the term "context" may be used interchangeably with the term "trinucleotide context", which is meant to include 1-bp nucleotides before and after the locus where the SNV occurs. It is known that the same SNVs also have different error rates depending on the context, and one type of SNV is classified into 16 context errors. For example, in the case of an A>T variant, a total of 192 errors (4 x 12 x 4) may appear depending on the types of nucleotides before and after the reference allele A, and each error rate differs between variants as shown in FIG 2.

Finally, step e) of determining the presence or absence of a false positive variant from the LLRc value obtained in step d) is performed.

According to one embodiment of the present invention, step e) of determining the presence or absence of a false positive variant may comprise: obtaining a weighted LLR value by calculating an LLR value for SSCS and an LLR value for DCS; and determining that, when a cut-off value set using a precision-recall curve from the weighted LLR value is 50 or more, the variant in the nucleic acid fragment containing the candidate variant is false positive.

**Mode for Invention**

Hereinafter, one or more embodiments will be described in more detail with reference to examples. However, these examples are for explaining one or more embodiments in detail, and the scope of the present invention is not limited to these examples.

**Example 1: Examination of error rate versus family size**

Three healthy person cfDNA samples (120 Gbp in total), four lung cancer patient-derived cfDNA samples (350 Gbp in total), cfDNA mixture 1 (composed of 5 colon cancer patients; 4 replicates; 221 Gbp in total), cfDNA mixture 2 (composed of 5 colon cancer patients; 4 replicates; 216 Gbp in total), and cfDNA mixture 3 (composed of 4 gastric cancer patients and 3 colon cancer patients; 26 replicates; 836 Gbp in total) were randomly subsampled to obtain 420 healthy person cfDNA subsamples (8,400 Gbp in total), 380 lung cancer patient-derived cfDNA subsamples (10,050 Gbp in total), 253 cfDNA mixture 1 subsamples (5,090 Gbp in total), 253 cfDNA mixture 2 subsamples (5,090 Gbp in total), and 1,380 cfDNA mixture 3 subsamples (21,900 Gbp in total), which were then used in error rate calculation. Sample types and downsampling conditions used in this Example are shown in Table 1.

EP 4 506 465 A1

[Table 1]

| Specimen label | Replicate number | The number of subsamples by data size | | | | | | | | | | Total Count | Total Data Size (Gbp) | Total subsample count | Total Subsample Data Size (Gbp ) |
|---|---|---|---|---|---|---|---|---|---|---|---|---|---|---|---|
| | | 5 Gbp | 10 Gbp | 15 Gbp | 20 Gbp | 25 Gbp | 30 Gbp | 35 Gbp | 40 Gbp | 45 Gbp | 50 Gbp | | | | |
| Healthy person cfDNA 1 | #1 | 20 | 20 | 20 | 20 | 20 | 20 | 20 | - | - | - | 140 | 2800 | 420 | 8,400 |
| Healthy person cfDNA 2 | #1 | 20 | 20 | 20 | 20 | 20 | 20 | 20 | - | - | - | 140 | 2800 | | |
| Healthy person cfDNA 3 | #1 | 20 | 20 | 20 | 20 | 20 | 20 | 20 | - | - | - | 140 | 2800 | | |
| Lung cancer cfDNA 1 | #1 | 10 | 10 | 10 | 10 | 10 | 10 | 10 | 10 | 10 | 10 | 100 | 2750 | 380 | 10,05 0 |
| Lung cancer cfDNA 2 | #1 | 10 | 10 | 10 | 10 | 10 | 10 | 10 | 10 | 10 | 10 | 100 | 2750 | | |
| Lung cancer cfDNA 3 | #1 | 10 | 10 | 10 | 10 | 10 | 10 | 10 | 10 | 10 | 10 | 100 | 2750 | | |
| Lung cancer cfDNA 4 | #1 | 10 | 10 | 10 | 10 | 10 | 10 | 10 | 10 | - | - | 80 | 1800 | | |
| Patient cfDNA mixture 1 (5 colon cancer patients) | #1 | 10 | 10 | 10 | 10 | 10 | 1 | - | - | - | - | 51 | 780 | 253 | 5,090 |
| | #2 | 10 | 10 | 10 | 10 | 10 | 1 | - | - | - | - | 51 | 780 | | |
| | #3 | 10 | 10 | 10 | 10 | 10 | 1 | - | - | - | - | 51 | 780 | | |
| | #4 | 10 | 10 | 10 | 10 | 10 | 10 | 10 | 10 | 10 | 10 | 100 | 2750 | | |
| Patient cfDNA mixture 2 (5 colon cancer patients) | #1 | 10 | 10 | 10 | 10 | 10 | 1 | - | - | - | - | 51 | 780 | 253 | 5,090 |
| | #2 | 10 | 10 | 10 | 10 | 10 | 1 | - | - | - | - | 51 | 780 | | |
| | #3 | 10 | 10 | 10 | 10 | 10 | 1 | - | - | - | - | 51 | 780 | | |
| | #4 | 10 | 10 | 10 | 10 | 10 | 10 | 10 | 10 | 10 | 10 | 100 | 2750 | | |
| Patient cfDNA mixture 3 (4 gastric cancer patients + 3 colon cancer patients) | #1 | 10 | 10 | 10 | 10 | 10 | 1 | - | - | - | - | 51 | 780 | 1,380 | 21,90 0 |
| | #2 | 10 | 10 | 10 | 10 | 10 | 1 | - | - | - | - | 51 | 780 | | |
| | #3 | 10 | 10 | 10 | 10 | 10 | 1 | - | - | - | - | 51 | 780 | | |
| | #4 | 10 | 10 | 10 | 10 | 10 | 1 | - | - | - | - | 51 | 780 | | |
| | #5 | 10 | 10 | 10 | 10 | 10 | 1 | - | - | - | - | 51 | 780 | | |
| | #6 | 10 | 10 | 10 | 10 | 10 | 1 | - | - | - | - | 51 | 780 | | |
| | #7 | 10 | 10 | 10 | 10 | 10 | 1 | - | - | - | - | 51 | 780 | | |

7

| Specimen label | Replicate number | The number of subsamples by data size | | | | | | | | | | Total Count | Total Data Size (Gbp) | Total subsample count | Total Subsample Data Size (Gbp ) |
|---|---|---|---|---|---|---|---|---|---|---|---|---|---|---|---|
| | | 5 Gbp | 10 Gbp | 15 Gbp | 20 Gbp | 25 Gbp | 30 Gbp | 35 Gbp | 40 Gbp | 45 Gbp | 50 Gbp | | | | |
| | #8 | 10 | 10 | 10 | 10 | 10 | 1 | - | - | - | - | 51 | 780 | | |
| | #9 | 10 | 10 | 10 | 10 | 10 | 1 | - | - | - | - | 51 | 780 | | |
| | #10 | 10 | 10 | 10 | 10 | 10 | 1 | - | - | - | - | 51 | 780 | | |
| | #11 | 10 | 10 | 10 | 10 | 10 | 1 | - | - | - | - | 51 | 780 | | |
| | #12 | 10 | 10 | 10 | 10 | 10 | 1 | - | - | - | - | 51 | 780 | | |
| | #13 | 10 | 10 | 10 | 10 | 10 | 1 | - | - | - | - | 51 | 780 | | |
| | #14 | 10 | 10 | 10 | 10 | 10 | 1 | - | - | - | - | 51 | 780 | | |
| | #15 | 10 | 10 | 10 | 10 | 10 | 1 | - | - | - | - | 51 | 780 | | |
| | #16 | 10 | 10 | 10 | 10 | 10 | 1 | - | - | - | - | 51 | 780 | | |
| | #17 | 10 | 10 | 10 | 10 | 10 | 1 | - | - | - | - | 51 | 780 | | |
| | #18 | 10 | 10 | 10 | 10 | 10 | 1 | - | - | - | - | 51 | 780 | | |
| | #19 | 10 | 10 | 10 | 10 | 10 | 1 | - | - | - | - | 51 | 780 | | |
| | #20 | 10 | 10 | 10 | 10 | 10 | 1 | - | - | - | - | 51 | 780 | | |
| | #21 | 10 | 10 | 10 | 10 | 10 | 10 | - | - | - | - | 60 | 1050 | | |
| | #22 | 10 | 10 | 10 | 10 | 10 | 10 | - | - | - | - | 60 | 1050 | | |
| | #23 | 10 | 10 | 10 | 10 | 10 | 10 | - | - | - | - | 60 | 1050 | | |
| | #24 | 10 | 10 | 10 | 10 | 10 | 10 | - | - | - | - | 60 | 1050 | | |
| | #25 | 10 | 10 | 10 | 10 | 10 | 10 | - | - | - | - | 60 | 1050 | | |
| | #26 | 10 | 10 | 10 | 10 | 10 | 10 | - | - | - | - | 60 | 1050 | | |

From the fastq file created by subsampling, the adapter sequence was removed using the fastp program, and the results were mapped to the human reference genome GRCh38 using the bwa MEM program and recorded in bam format. Then, the SSCS bam file and the DCS bam file were created using the fgbio program, and the bam files were divided by the family size according to the consensus depth (cD) tag value indicating the family size. In the above process, a part different from the reference genome was found in the bam file created for each family size, and the context type of the corresponding location was identified and the number of errors for each context was counted. At this time, for the entire test region (106 genes, 365,175 bp), the parts different from the reference genome sequence at the remaining positions except for the previously known positions of germ cell variants and somatic variants were determined as errors, and the error rate was calculated by counting the total frequency for each context type and dividing the count by the number of errors.

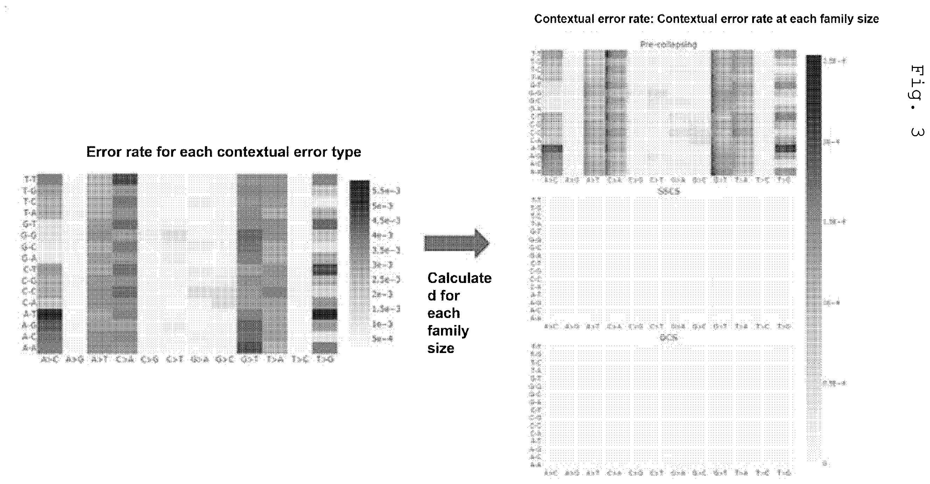

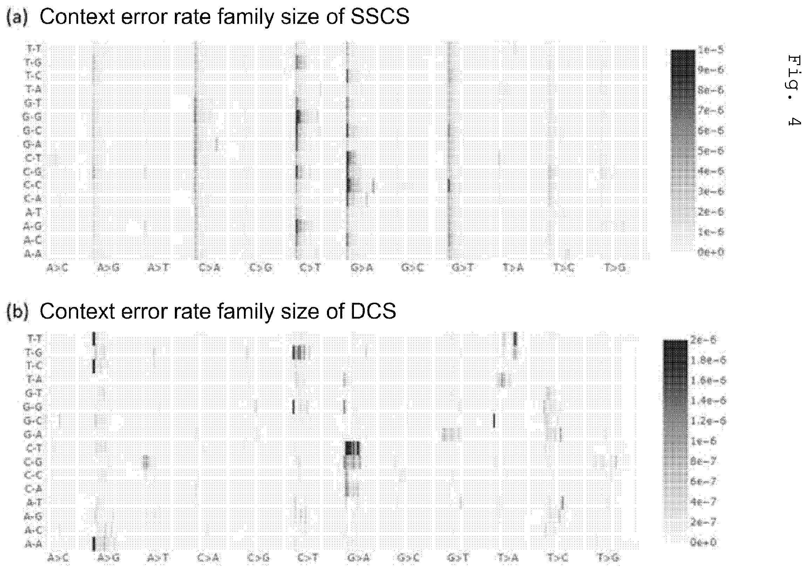

FIG. 3 shows error rates in color in a total of 29 different columns corresponding to a family size of 2 to 30 for each type of context. As a result, it could be confirmed that the error rate tended to gradually decrease as the family size increased. In addition, as a result of checking the context error rate for each of the SSCS and the DCS, as shown in FIG. 4, it was confirmed that the error rate was lower in the DCS than in the SSCS. Taking these results together, it could be seen that the error rate decreased as the family size increased.

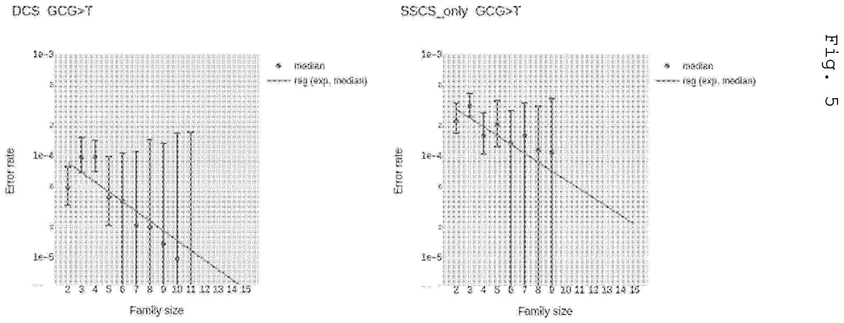

In addition, a probability density function was created by fitting the median values of the error rates observed for each family size to an exponential distribution, and the error rate versus the family size was calculated therefrom. In addition, since groups and contexts with large family sizes where almost no errors are observed have very low error rates, which can result in inaccurate measurements and statistical bias, the value was capped to $1e^{-10}$. As a result, as shown in FIG. 5, it could be confirmed that, in the case of the GCG>T context in which the error rate was high, the error rates at a family size of 2 were $2.54e^{-4}$ in the case of SSCS and $8.13e^{-5}$ in the case of DCS, which decreased as the family size increased, and the error rates at a family size of 7 were $1.06e^{-4}$ in the case of SSCS and $2.84e^{-5}$ in the case of DCS, indicating that the error rates tended to decrease gradually as the family size increased.

**Example 2: Examination of error rate depending on type of indel context for each family size**

Insertions and deletions have various lengths, and infinite combinations thereof are possible depending on the positions where variants occur. In this Example, for indel contexts, a total of 8,745 contexts were prepared by dividing references and variants into 75 and 113 categories, respectively, and were used to examine the error rate depending on the family size.

The reference category context is indicated as follows:

[unit]:[unit length]:[repeat count]

For example, if the reference is AA[TTTTT]AA, the context is denoted as T:1:5.

The variant category context is indicated as follows:

[unit length]:[variant type]:[unit]:[repeat count]

For example, if the reference is AA[TTTTT]AA and the variant is AA[TTT-]AA, the context is denoted as 1:Del:T:2.

Terms used in the above contexts and descriptions and examples thereof are shown in Table 2 below.

[Table 2]

| Term | Description | Value | Example |
|---|---|---|---|
| Unit | Indicates a repeat unit.<br>R: Repeats of 2 bp or longer are included regardless of nucleotide.<br>M : micro-homology<br>N : case in which the nucleotide of the reference sequence is unknown | N, A, T, G, C, R, M | If GC or AT is a repeat unit, all are included in the R category |
| Unit length | Length of repeat unit | 1 to 6 | GC is 2, and GCC is 3 |
| Repeat count | Number of repeats | 1 to 6 | GCGCGC corresponds to three GC repeats |
| Variant type | Type of variant | Ins, Del | |

Tables 3 and 4 below show 75 reference category contexts and 113 variant category contexts according to the definitions used in this Example.

[Table 3]

| Reference categories | | | | | | | | | | | |
|---|---|---|---|---|---|---|---|---|---|---|---|
| N:1:0 | A:1:1 | T:1:1 | G:1:1 | C:1:1 | R:2:1 | R:3:1 | R:4:1 | R:5:1 | R:6:1 | N:2:0 | A:1:2 |
| | T:1:2 | G:1:2 | C:1:2 | R:2:2 | R:3:2 | R:4:2 | R:5:2 | R:6:2 | | | |
| N:3:0 | A:1:3 | T:1:3 | G:1:3 | C:1:3 | R:2:3 | R:3:3 | R:4:3 | R:5:3 | R:6:3 | | |
| N:4:0 | A:1:4 | T:1:4 | G:1:4 | C:1:4 | R:2:4 | R:3:4 | R:4:4 | R:5:4 | R:6:4 | | |
| N:5:0 | A:1:5 | T:1:5 | G:1:5 | C:1:5 | R:2:5 | R:3:5 | R:4:5 | R:5:5 | R:6:5 | | |
| N:6:0 | A:1:6 | T:1:6 | G:1:6 | C:1:6 | R:2:6 | R:3:6 | R:4:6 | R:5:6 | R:6:6 | | |
| M:2:1 | M:3:1 | M:4:1 | M:5:1 | M:6:1 | | | | | | | |
| | M:3:2 | M:4:2 | M:5:2 | M:6:2 | | | | | | | |
| | | M:4:3 | M:5:3 | M:6:3 | | | | | | | |
| | | | M:5:4 | M:6:4 | | | | | | | |
| | | | | M:6:5 | | | | | | | |

[Table 4]

| Variant categories | | | | | | | |
|---|---|---|---|---|---|---|---|
| 1:Ins:A:1 | 1:Ins:T:1 | 1:Ins:G:1 | 1:Ins:C:1 | 1:Del:A:1 | 1:Del:T:1 | 1:Del:G:1 | 1:Del:C:1 |
| 1:Ins:A:2 | 1:Ins:T:2 | 1:Ins:G:2 | 1:Ins:C:2 | 1:Del:A:2 | 1:Del:T:2 | 1:Del:G:2 | 1:Del:C:2 |
| 1:Ins:A:3 | 1:Ins:T:3 | 1:Ins:G:3 | 1:Ins:C:3 | 1:Del:A:3 | 1:Del:T:3 | 1:Del:G:3 | 1:Del:C:3 |
| 1:Ins:A:4 | 1:Ins:T:4 | 1:Ins:G:4 | 1:Ins:C:4 | 1:Del:A:4 | 1:Del:T:4 | 1:Del:G:4 | 1:Del:C:4 |
| 1:Ins:A:S | 1:Ins:T:5 | 1:Ins:G:5 | 1:Ins:C:5 | 1:Del:A:5 | 1:Del:T:5 | 1:Del:G:5 | 1:Del:C:5 |
| 1:Ins:A:6 | 1:Ins:T:6 | 1:Ins:G:6 | 1:Ins:C:6 | 1:Del:A:6 | 1:Del:T:6 | 1:Del:G:6 | 1:Del:C:6 |
| 2:Ins:R:1 | 2:Ins:R:2 | 2:Ins:R:3 | 2:Ins:R:4 | 2:Ins:R:5 | 2:Ins:R:6 | | |
| 3:Ins:R:1 | 3:Ins:R:2 | 3:Ins:R:3 | 3:Ins:R:4 | 3:Ins:R:5 | 3:Ins:R:6 | | |
| 4:Ins:R:1 | 4:Ins:R:2 | 4:Ins:R:3 | 4:Ins:R:4 | 4:Ins:R:5 | 4:Ins:R:6 | | |
| 5:Ins:R:1 | 5:Ins:R:2 | 5:Ins:R:3 | 5:Ins:R:4 | 5:Ins:R:5 | 5:Ins:R:6 | | |
| 6:Ins:R:1 | 6:Ins:R:2 | 6:Ins:R:3 | 6:Ins:R:4 | 6:Ins:R:5 | 6:Ins:R:6 | | |
| 2:Del:R:1 | 2:Del:R:2 | 2:Del:R:3 | 2:Del:R:4 | 2:Del:R:5 | 2:Del:R:6 | | |
| 2:Del:M:0 | | | | | | | |
| 3:Del:R:1 | 3:Del:R:2 | 3:Del:R:3 | 3:Del:R:4 | 3:Del:R:5 | 3:Del:R:6 | | |
| 3:Del:M:0 | | | | | | | |
| 4:Del:R:1 | 4:Del:R:2 | 4:Del:R:3 | 4:Del:R:4 | 4:Del:R:5 | 4:Del:R:6 | | |
| 4:Del:M:0 | | | | | | | |
| 5:Del:R:1 | 5:Del:R:2 | 5:Del:R:3 | 5:Del:R:4 | 5:Del:R:5 | 5:Del:R:6 | | |
| 5:Del:M:0 | | | | | | | |
| 6:Del:R:1 | 6:Del:R:2 | 6:Del:R:3 | 6:Del:R:4 | 6:Del:R:5 | 6:Del:R:6 | | |
| 6:Del:M:0 | | | | | | | |

* In the above tables, micro homology (M) is not measurement of repetition of repeat units unlike other categories, and thus the count is indicated as 0.

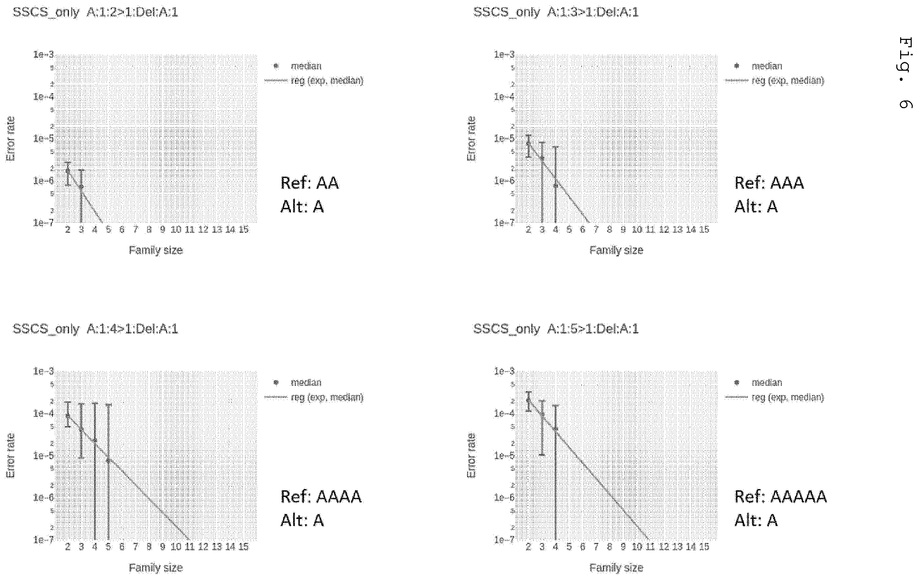

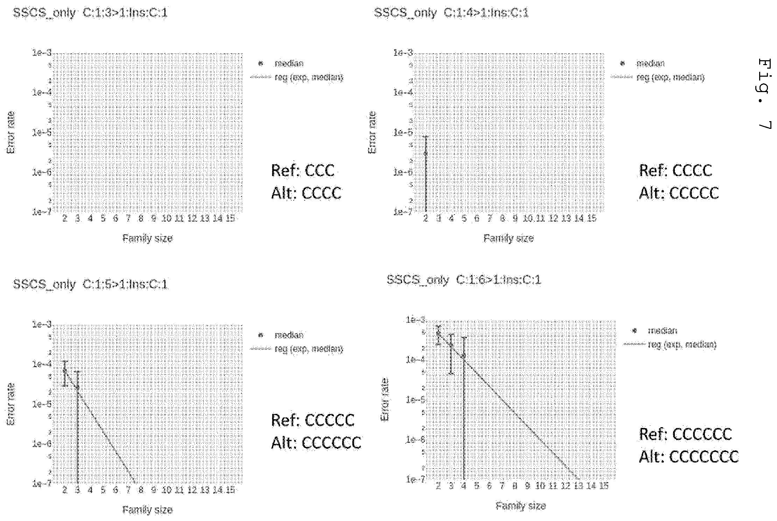

As can be seen in FIGS. 6 and 7, it could be confirmed that, in the case of deletion variants and insertion variants, the error rate significantly decreased the family size increased. In particular, in the case of deletion variants and insertion variants, the error rate increased as the length of the variant decreased, and as shown in FIG. 6, when the deletion length became long, there was also a context that rarely occurs. In addition, as shown in FIG. 7, it could be confirmed that the error rate was higher in the context with a longer repeat sequence.

**Example 3: Calculation of variant score**

The variant score for determining false positives was calculated using the log likelihood ratio (LLR). The LLR value for context (LLRc) was calculated according to the following Equation I.

[Equation I]

$$LLR_C = \sum_{r=1}^{N} ln\left(\frac{f \times (1-H) + (1-f) \times H}{H}\right) \qquad H = \begin{cases} e_{r(s),c}, & if\ r = alt\ read \\ 1 - e_{r(s),c}, & otherwise \end{cases}$$

wherein r denotes read, N denotes the total number of reads, S denotes the family size, f denotes variant allele frequency, and e denotes error rate.

The final variant score was calculated by comprehensively considering the following three error rates:

1) the context error rate at a specific family size;
2) the nucleotide error rate calculated in the error correction process; and
3) the read error rate calculated in the mapping process.

Each LLR value obtained by substituting the above three error rates into Equation I is as follows.

1) CXT_LLR$_{DCS}$: the sum of the LLR values calculated by the DCS context error rates at all family sizes;
2) CXT_LLR$_{SSCS}$: the sum of the LLR values calculated by the SSCS context error rates at all family sizes;
3) BQ_LLR$_{DCS}$: the sum of the LLR values calculated by the nucleotide error rates calculated in the DCS error correction process at all family sizes;
4) BQ_LLR$_{SSCS}$: the sum of the LLR values calculated by the nucleotide error rates calculated in the SSCS error correction process at all family sizes;
5) MQ_LLR$_{DCS}$: the sum of the LLR values calculated by the read error rates calculated in the DCS mapping process at all family sizes; and
6) MQ_LLR$_{SSCS}$: the sum of the LLR values calculated by the read error rates calculated in the SSCS mapping process at all family sizes.

The LLRs may be summarized as follows:

$$LLR_{DCS} = CXT\_LLR_{DCS} + BQ\_LLR_{DCS} + MQ\_LLR_{DCS}$$

$$LLR_{SSCS} = CXT\_LLR_{SSCS} + BQ\_LLR_{SSCS} + MQ\_LLR_{SSCS}$$

The weighted LLR value (wLLR) for calculating the final variant score is calculated as follows.

$$wLLR = (2 \times LLR_{DCS} + LLR_{SSCS})/3$$

The cut-off of scores for determining false positives was set using a precision-recall curve.

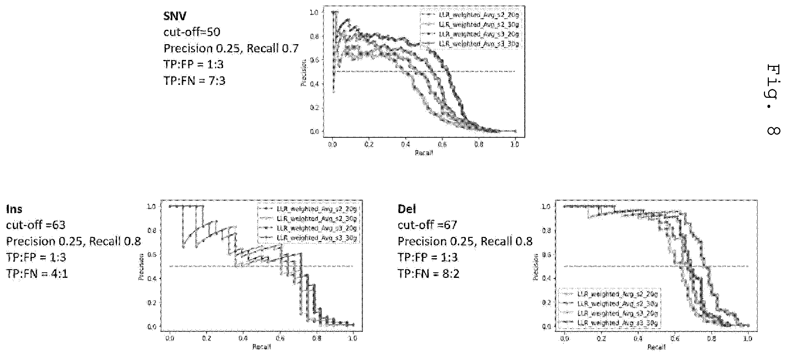

In the NGS test, false positives can be further removed through other measurement criteria, but elimination of false negatives leads to worse results, and thus the cutoff was set at a precision of 0.25 and a recall of 0.7 to 0.8. In the error correction step, two types of parameters were set according to the error processing strength (s2 and s3), and analysis was performed under two data amount conditions (20 Gbp and 30 Gbp). As a result, as shown in FIG. 8, it could be confirmed that, in the case of the SNV, the performance was the best under the conditions of s3 and 30 Gbp, and in the case of the insertion variant, the performance was not significantly affected by the conditions, and in the case of the deletion variant, the performance was excellent under the conditions of s2 and 30 Gbp.

So far, the present invention has been described with reference to the preferred embodiments. Those skilled in the art will appreciate that the present invention can be implemented in modified forms without departing from the essential features of the present invention. Therefore, the disclosed embodiments should be considered in descriptive sense only and not for purposes of limitation. Therefore, the scope of the present invention is defined not by the detailed description of the present invention but by the appended claims, and all differences within a range equivalent to the scope of the appended claims should be construed as being included in the present invention.

**Claims**

1. A method of removing false positive variants in nucleic acid sequencing, the method comprising steps of:

   a) extracting a nucleic acid fragment containing a candidate variant from a target sample;
   b) adding a unique molecular identifier (UMI) to an end of the extracted nucleic acid fragment;
   c) producing a high-quality unique sequence (HQS) by amplifying the nucleic acid fragment to which the UMI has been added;
   d) obtaining an LLRc value according to the following Equation I by applying an error rate corresponding to the HQS; and
   e) determining the presence or absence of a false positive variant from the LLRc value:

   [Equation I]

   $$LLR_C = \sum_{r=1}^{N} ln\left(\frac{f \times (1-H) + (1-f) \times H}{H}\right) \qquad H = \begin{cases} e_{r(S),C}, & if\ r = alt\ read \\ 1 - e_{r(S),C}, & otherwise \end{cases}$$

   wherein r denotes read, N denotes total number of reads, S denotes family size, f denotes variant allele frequency, and e denotes error rate.

2. The method according to claim 1, wherein the candidate variant is at least one selected from the group consisting of single-nucleotide variation, nucleotide insertion, and nucleotide deletion.

3. The method according to claim 1, wherein the HQS in step c) is a single-strand consensus sequence (SSCS) or a duplex consensus sequence (DCS).

4. The method according to claim 1, wherein the family size is 2 to 30.

5. The method according to claim 1, wherein the error rate includes all of a context error rate at a specific family size, a nucleotide error rate calculated in an error correction process, and a read error rate calculated in a mapping process.

6. The method according to claim 1, wherein step e) of determining the presence or absence of the false positive variant comprises: obtaining a weighted LLR value by calculating an LLR value for SSCS and an LLR value for DCS; and determining that, when a cut-off value set using a precision-recall curve from the weighted LLR value is 50 or more, the variant in the nucleic acid fragment containing the candidate variant is false positive.

Fig. 1

Fig. 2

(b) Error rate for each contextual error type

(a) 192 contextual error types

12 SNV types

Fig. 3

Fig. 4

(a) Context error rate family size of SSCS

(b) Context error rate family size of DCS

Fig. 5

Fig. 6

Fig. 7

Fig. 8

**SNV**
cut-off=50
Precision 0.25, Recall 0.7
TP:FP = 1:3
TP:FN = 7:3

**Del**
cut-off =67
Precision 0.25, Recall 0.8
TP:FP = 1:3
TP:FN = 8:2

**Ins**
cut-off =63
Precision 0.25, Recall 0.8
TP:FP = 1:3
TP:FN = 4:1

| INTERNATIONAL SEARCH REPORT | International application No. |
|---|---|
| | **PCT/KR2022/011529** |

**A. CLASSIFICATION OF SUBJECT MATTER**

**C12Q 1/6827**(2018.01)i; **C12Q 1/6886**(2018.01)i

According to International Patent Classification (IPC) or to both national classification and IPC

**B. FIELDS SEARCHED**

Minimum documentation searched (classification system followed by classification symbols)

C12Q 1/6827(2018.01); C12Q 1/6806(2018.01); C12Q 1/6869(2018.01); G16B 20/20(2019.01); G16B 25/10(2019.01); G16B 30/00(2019.01)

Documentation searched other than minimum documentation to the extent that such documents are included in the fields searched

Korean utility models and applications for utility models: IPC as above
Japanese utility models and applications for utility models: IPC as above

Electronic data base consulted during the international search (name of data base and, where practicable, search terms used)

eKOMPASS (KIPO internal) & keywords: 핵산 서열(nucleic acid), 위양성 변이(false positive variants), 고유 분자 식별자 (unique molecular identifier, UMI), HQS(high quality unique sequence), 오류율(error rate), 대립유전자 빈도(variant allele frequency)

**C. DOCUMENTS CONSIDERED TO BE RELEVANT**

| Category* | Citation of document, with indication, where appropriate, of the relevant passages | Relevant to claim No. |
|---|---|---|
| A | HUANG, C.-C. et al. Bioinformatics Analysis for Circulating Cell-Free DNA in Cancer. Cancers. 2019, vol. 11, thesis no. 805, pp. 1-15.<br>See abstract; pages 2-3; and figures 1-2. | 1-6 |
| A | KR 10-2020-0013709 A (ILLUMINA, INC.) 07 February 2020 (2020-02-07)<br>See claims 1-19; and paragraphs [0038] and [0047]-[0049]. | 1-6 |
| A | WANG, T. T. et al. High efficiency error suppression for accurate detection of low-frequency variants. Nucleic Acids Research. 2019, vol. 47, no. 15, thesis no. e87, pp. 1-11.<br>See abstract; pages 3-4; and figure 1. | 1-6 |
| A | KR 10-2021-0040714 A (GENINUS INC.) 14 April 2021 (2021-04-14)<br>See claims 1-14. | 1-6 |
| A | CN 111304288 A (JIANGSU XIANSHENG MEDICAL DIAGNOSIS CO., LTD. et al.) 19 June 2020 (2020-06-19)<br>See entire document. | 1-6 |

☐ Further documents are listed in the continuation of Box C.  ☑ See patent family annex.

| * | Special categories of cited documents: | "T" | later document published after the international filing date or priority date and not in conflict with the application but cited to understand the principle or theory underlying the invention |
|---|---|---|---|
| "A" | document defining the general state of the art which is not considered to be of particular relevance | | |
| "D" | document cited by the applicant in the international application | "X" | document of particular relevance; the claimed invention cannot be considered novel or cannot be considered to involve an inventive step when the document is taken alone |
| "E" | earlier application or patent but published on or after the international filing date | | |
| "L" | document which may throw doubts on priority claim(s) or which is cited to establish the publication date of another citation or other special reason (as specified) | "Y" | document of particular relevance; the claimed invention cannot be considered to involve an inventive step when the document is combined with one or more other such documents, such combination being obvious to a person skilled in the art |
| "O" | document referring to an oral disclosure, use, exhibition or other means | "&" | document member of the same patent family |
| "P" | document published prior to the international filing date but later than the priority date claimed | | |

| Date of the actual completion of the international search | Date of mailing of the international search report |
|---|---|
| **01 December 2022** | **02 December 2022** |

| Name and mailing address of the ISA/KR | Authorized officer |
|---|---|
| **Korean Intellectual Property Office**<br>**Government Complex-Daejeon Building 4, 189 Cheongsa-ro, Seo-gu, Daejeon 35208** | |
| Facsimile No. **+82-42-481-8578** | Telephone No. |

Form PCT/ISA/210 (second sheet) (July 2022)

| | International application No. |
|---|---|
| | **PCT/KR2022/011529** |

| Patent document cited in search report | | | Publication date (day/month/year) | Patent family member(s) | | | Publication date (day/month/year) |
|---|---|---|---|---|---|---|---|
| KR | 10-2020-0013709 | A | 07 February 2020 | AU | 2018-375785 | A1 | 12 December 2019 |
| | | | | AU | 2021-269294 | A1 | 09 December 2021 |
| | | | | CA | 3067425 | A1 | 06 June 2019 |
| | | | | CN | 110870016 | A | 06 March 2020 |
| | | | | EP | 3718113 | A1 | 07 October 2020 |
| | | | | JP | 2020-524499 | A | 20 August 2020 |
| | | | | JP | 7013490 | B2 | 15 February 2022 |
| | | | | KR | 10-2356323 | B1 | 26 January 2022 |
| | | | | US | 2019-0206510 | A1 | 04 July 2019 |
| | | | | WO | 2019-108972 | A1 | 06 June 2019 |
| KR | 10-2021-0040714 | A | 14 April 2021 | KR | 10-2347463 | B1 | 06 January 2022 |
| CN | 111304288 | A | 19 June 2020 | | None | | |

Form PCT/ISA/210 (patent family annex) (July 2022)